(19) Europäisches Patentamt
European Patent Office
Office européen des brevets

(11) Publication number: **0 532 506 B1**

# (12) EUROPEAN PATENT SPECIFICATION

(45) Date of publication of patent specification: **18.10.95**

(51) Int. Cl.⁶: **C07D 409/12**, A61K 31/40

(21) Application number: **91907069.8**

(22) Date of filing: **02.04.91**

(86) International application number:
**PCT/EP91/00622**

(87) International publication number:
**WO 91/18896 (12.12.91 91/28)**

The file contains technical information submitted after the application was filed and not included in this specification

## (54) 4-[(Thiophene-2-carbonyl)oxy]pyrrolidine-2-carboxylic acid, its preparation and its use as anti-inflammatory and anti-dystrophic agent.

(30) Priority: **30.05.90 IT 2048390**

(43) Date of publication of application:
**24.03.93 Bulletin 93/12**

(45) Publication of the grant of the patent:
**18.10.95 Bulletin 95/42**

(84) Designated Contracting States:
**AT BE CH DE DK ES FR GB GR IT LI LU NL SE**

(56) References cited:
**DE-A- 2 139 476**
**FR-A- 2 152 456**

**Chemical Abstracts, File/handbook, CN 129336-81-8 "Tenosiprol"**

(73) Proprietor: **PULITZER ITALIANA S.r.l.**
**Via Tiburtina, 1004**
**I-00156 Roma (IT)**

(72) Inventor: **RAINOLDI, Angelo**
**Via Tiburtina, 1004**
**I-00156 Rome (IT)**

(74) Representative: **Bianchetti, Giuseppe**
**Studio Consulenza Brevettuale,**
**Via Rossini, 8**
**I-20122 Milan (IT)**

EP 0 532 506 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to 4-(thiophene-2-carbonyl)-oxypyrrolidine-2-carboxylic acid, of formula I

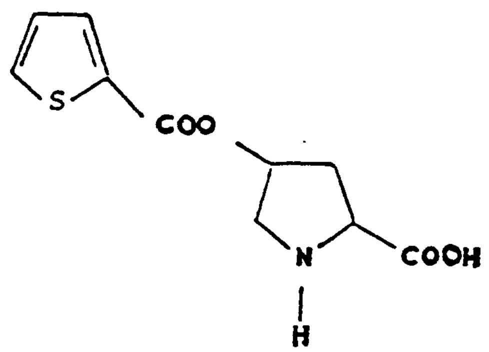

(I)

and to the salts thereof with pharmaceutically acceptable acids or bases.

The present invention also relates to a process for the preparation of compound I as well as to the pharmaceutical compositions containing it as the active ingredient. Examples of pharmaceutically acceptable salts are the hydrochloride, sulfate, citrate, tartrate, p-toluenesulphonate salts as well as the sodium, potassium, calcium, magnesium, ammonium, triethylammonium salts.

N-acetyl-hydroxyproline is an amino acid already used in therapy due to its cutis healing and eutrophizing activities and anti-inflammatory activity.

FR-A-2.152.456 discloses O-lipoyl, N-lipoyl and N,O-dilipoyl derivatives of hydroxyproline particularly useful as topical antiinflammatory agents.

Now it has been found that compound I remarkably improves the pharmacological and pharmacokinetic characteristics of the N-acetyl derivative cited above.

Therefore, another object of the present invention is provided by pharmaceutical compositions containing compound of formula I or a salt thereof in admixture with suitable carriers. The compositions of the invention can be used by the oral, parenteral or topical routes, for the treatment of a variety of diseases due to inflammatory and dystrophic conditions, such as arthrosis, dermatosclerosis, dystrophic vaginitis, skin and mucosa sores or ulcerations.

Examples of pharmaceutical formulations comprise capsules, tablets, drinkable solutions, syrups, vaginal suppositories, creams, suppositories, containing 1 to 10% active ingredient or unitary doses from 100 to 500 mg. The daily dosage will depend upon a number of factors, but generally it will range from 100 to 1000 mg daily, in one or more administrations.

Compound I is prepared by reacting hydroxyproline, previously suitably protected at the pyrrolidine nitrogen atom, with a reactive derivative of 2-thiophenecarboxylic acid, according to conventional procedures.

The following example further illustrates the invention.

**EXAMPLE**

a) 6,55 a (0.05 mole) of (L)-4-hydroxyproline are added to a 1M NaOH solution (NaOH 2.2 g; 0.055 mole in 55 ml of water). A solution of 10.9 g (0.05 mole) of di-tert-butyl dicarbonate in 20 ml of t-butanol is slowly dropped into the above solution (the reaction is slowly exothermic). The reaction mixture is left to react at room temperature under stirring for 18 hours. t-Butanol is evaporated off without exceeding 30 °C. The remained aqueous solution is cooled to 0-5 °C and acidified to pH = 1-1.5 by adding a solution of 11.3 g (0.0825 mole) of $KHSO_4$ in 75 ml of water : $CO_2$ development takes place. The solution is extracted with 3 x 20 ml of ethyl ether. The ether extracts are dried over $Na_2SO_4$ and evaporated, to obtain the 4-hydroxyproline protected derivative (A) as a colourless solid. Yield : 9.2 g (80%). m.p. : 120 °C (lit. 134 °C). N.M.R. ($D_2O$): in conformity.

b) A mixture of 10 g (0.078 mole) of 2-thiophenecarboxylic acid and 30 ml of thionyl chloride is refluxed for a night; then thionyl chloride is evaporated off and the residue is distilled in Vigreux under reduced pressure : T = 128-130 °C; P = 2.5325 X $10^4$ Pa (190 mmHg)

2-Thiophenecarboxylic acid chloride (B) is obtained; yield : 9.06 g (79%).

c) 8.5 g (0.0368 mole) at product (A) are dissolved in 18 ml of 10% NaOH. The solution is cooled to 0 °C and a solution of 5.93 g (0.0368 mole) of product (B) in 5 ml of dioxane is dropped therein, then 18 ml of 10% NaOH, keeping the same temperature. When the additions are completed, the reaction mixture is left to warm to room temperature and to react overnight, then it is acidified to pH 4 with concentrated hydrochloric acid and extracted with ethyl ether. The extract is dried over $Na_2SO_4$ and evaporated, the obtained viscous residue (11.5 g) is subjected to chromatography on 200 g of silica gel, using a dioxane/toluene/acetic acid 10:45:2 mixture as the eluent, to separate the desired product (C) (having $R_f$ = 0.4) from unreacted 2-thiophenecarboxylic acid (having $R_f$ = 0.5).

9.17 g of a glassy residue are obtained, which consists of (C) and, to a small extent, of 2-thiophenecarboxylic acid, which is used directly.

A small amount is treated with n-hexane/diisopropyl ether 10:1, to obtain (C) as a colourless solid. Yield : 9.17 g (73%); m.p. 129-131 °C; T.L.C. : (toluene/dioxane/acetic acid = 10:45:2); unitary, $R_f$ =0.4; $[\alpha]_D^{20}$ = -47.4 ° (C = 1, $CH_3OH$); N.M.R. ($CDCl_3$) : in conformity.

d) 8.32 g (0.0244 mole) of (C) are dissolved in 10 ml of anhydrous tetrahydrofuran, then 35 ml of 3.9M HCl in THF (equivalent to 0.0244 x 5 mole) are added thereto. The reaction mixture is left to react at room temperature overnight, with stirring. Since T.L.C. checking proves that the reaction is not completed, 10 ml more of 3.9 M HCl in THF are added, and the mixture is left to react for 7 hours more. The reaction proceeds but it is not still completed : 5 ml of 3.9M HCl in THF are added and the mixture is left to react for 15 hours until the reaction is completed. Solvent is evaporated off and the solid colourless residue is taken up into ethyl ether (in which 2-dodecenecarboxylic acid is soluble) and filtered.

The hydrochloride of the compound is obtained, in form of a colourless solid. Yield : 5.46 g (81%); m.p. 248-250 °C (the product becomes dark). T.L.C. : (eluent : toluene/dioxane/AcOH = 10/45/2) : unitary.
$[\alpha]_D^{20}$ : + 2.4 ° (c = 0,5 $CH_3OH$)

- I.R. (nujol):
  1700 $cm^{-1}$ $\nu C=O$ -COOH
  1745 $cm^{-1}$ $\nu C=O$ -COO-R
- elemental analysis (for $C_{10}H_{12}ClNO_4S$)

| | | | |
|---|---|---|---|
| % calc. | C 43.24 | H 4.32 | N 5.05 |
| % found | C 43.13 | H 4.26 | N 5.18 |

- NMR (DMSO) δ ppm: 2.4-2.6 (m, 2H); 3.3-3.8 (m, 2H); 4.4-4.6 (d,d 1H); 5.5 (m, 1H); 7.2 (t, 1H); 7.9-8.1 (m, 2H); 10-11 (broad) (2H).

The signal at 10-11 δ disappears upon deuteration with $D_2O$.

## Claims

**Claims for the following Contracting States : AT, BE, CH, DE, DK, FR, GB, IT, LU, NL, SE**

**1.** 4-(Thiophene-2-carbonyl)-oxypyrrolidine-2-carboxylic acid of formula I

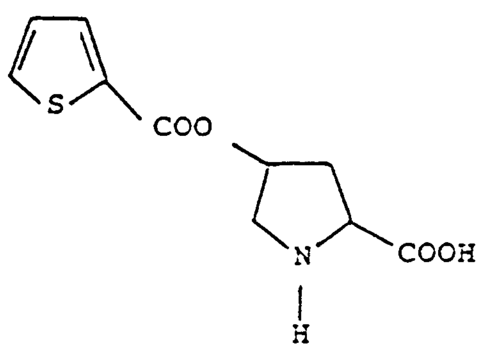

(I)

and the salts thereof with pharmaceutically acceptable acids or bases.

**2.** As a compound as claimed in claim 1, the hydrochloride of compound I.

3. A process for the preparation of compound I, in which 4-hydroxyproline, having the pyrrolidine nitrogen atom protected, is reacted with a reactive derivative of 2-thiophenecarboxylic acid.

4. Pharmaceutical compositions containing a compound as claimed in claims 1-2 as the active ingredient, together with pharmaceutically acceptable carriers and/or excipients.

5. The use of a compound as claimed in claims 1-2 for the preparation of a medicament having anti-inflammatory and antidystrophic activities.

**Claim for the following Contracting States : ES, GR**

1. A process for the preparation of 4-(thiophene-2-carbonyl)-oxypyrrolidine-2-carboxylic acid of formula I

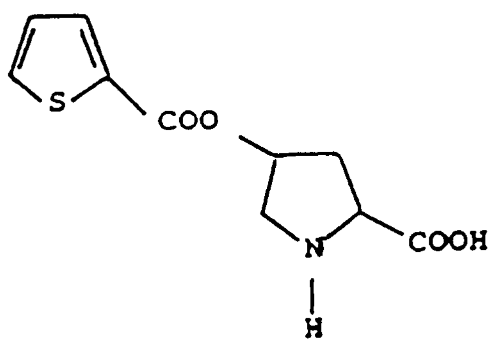

(I)

in which 4-hydroxyproline, having the pyrrolidine nitrogen atom protected, is reacted with a reactive derivative of 2-thiophenecarboxylic acid,
and the salts thereof with pharmaceutically acceptable acids or bases.

**Patentansprüche**
**Patentansprüche für folgende Vertragsstaaten : AT, BE, CH, DE, DK, FR, GB, IT, LU, NL, SE**

1. 4-(Thiophen-2-carbonyl)-oxypyrrolidin-2-carbonsäure der Formel I

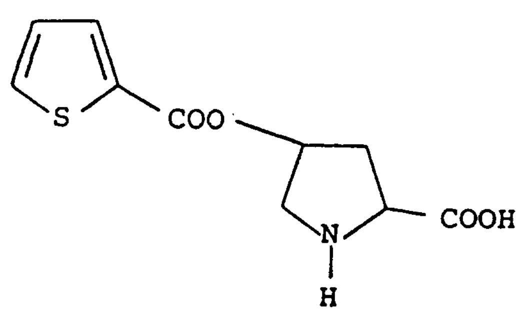

(I)

und Salze davon mit pharmazeutisch verträglichen Säuren oder Basen.

2. Als Verbindung nach Anspruch 1 das Hydrochlorid der Verbindung I.

3. Verfahren zur Herstellung von Verbindung I, bei dem 4-Hydroxyprolin, bei dem das Pyrrolidinstickstoffatom geschützt ist, mit einem reaktiven Derivat von 2-Thiophencarbonsäure umgesetzt wird.

4. Pharmazeutische Zusammensetzungen, enthaltend eine Verwendung nach Ansprüchen 1 - 2 als den Wirkstoff, zusammen mit pharmazeutisch verträglichen Trägern und/oder Excipientien.

5. Verwendung einer Verbindung nach Ansprüchen 1 - 2 zur Herstellung eines Medikaments, das antiinflammatorische und antidystrophische Wirkungen aufweist.

**Patentanspruch für folgende Vertragsstaaten : ES, GR**

1. Verfahren zur Herstellung von 4-(Thiophen-2-carbonyl)oxypyrrolidin-2-carbonsäure der Formel I

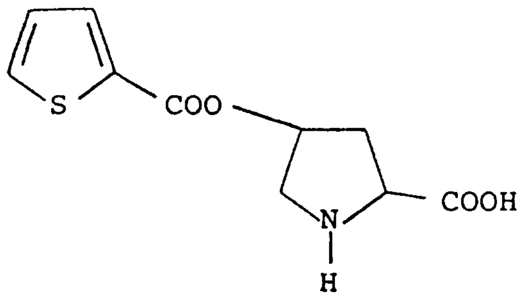

(I)

bei dem 4-Hydroxyprolin, bei dem das Pyrrolidinstickstoffatom geschützt ist, mit einem reaktiven Derivat von 2-Thiophencarbonsäure umgesetzt wird, und Salze davon mit pharmazeutisch verträglichen Säuren oder Basen.

**Revendications**
**Revendications pour les Etats contractants suivants : AT, BE, CH, DE, DK, FR, GB, IT, LU, NL, SE**

1. Acide 4-(thiophène-2-carbonyl)-oxypyrrolidine-2-carboxylique correspondant à la formule I

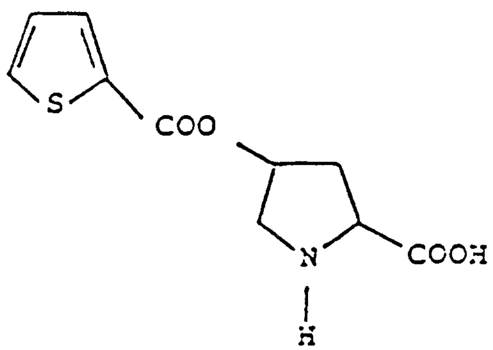

(I)

et ses sels formés avec des acides ou des bases acceptables du point de vue pharmaceutique.

2. Composé tel que revendiqué dans la revendication 1, qui est le chlorhydrate du composé I.

3. Procédé de préparation du composé I, dans lequel on fait réagir de la 4-hydroxyproline, ayant l'atome d'azote du groupe pyrrolidine protégé, avec un dérivé réactif de l'acide 2-thiophènecarboxylique.

4. Compositions pharmaceutiques contenant un composé tel que revendiqué dans les revendications 1 et 2 comme ingrédient actif, avec des véhicules et/ou des excipients acceptables du point de vue pharmaceutique.

5. Utilisation d'un composé tel que revendiqué dans les revendications 1 et 2 pour la préparation d'un médicament ayant des activités anti-inflammatoire et antidystrophique.

**Revendication pour les Etats contractants suivants : ES, GR**

1. Un procédé de préparation de l'acide 4-(thiophène-2-carbonyl)-oxypyrrolidine-2-carboxylique correspondant à la formule I

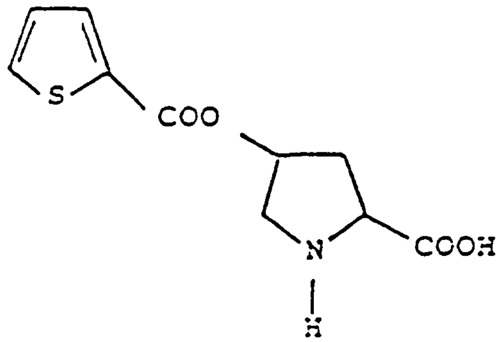

(I)

dans lequel on fait réagir de la 4-hydroxyproline, ayant l'atome d'azote du groupe pyrrolidine protégé, avec un dérivé réactif de l'acide 2-thiophènecarboxylique, et de ses sels formés avec des acides ou des bases acceptables du point de vue pharmaceutique.